# EUROPEAN PATENT APPLICATION

(11) **EP 3 124 597 A1**
(43) Date of publication of application: **01.02.2017**
(21) Application number: 15382395.0
(22) Date of filing: 29.07.2015
(51) Int. Cl.: C12N 1/14, C12P 23/00, A23L 5/46, C12R 1/645

(54) **A SPOROBOLOMYCES ROSEUS STRAIN FOR THE PRODUCTION OF COMPOSITIONS WITH COLORANT AND ANTIOXIDANT PROPERTIES**

(71) Applicant: Fundacíon Tecnalia Research & Innovation, 20009 Donostia - San Sebastian (ES); Centro Nacional de Technología y Seguridad alimentaria (CNTA), 31570 San Adrián (ES); Centro Tecnológico Agroalimentario Extremadura, 06195 Villafranco del Guadiana (ES)
(72) Inventor: MARAÑÓN GARCÍA, Izaskun, 20009 DONOSTIA - SAN SEBASTIÁN (ES); SAN VICENTE LAURENT, Leire, 20009 DONOSTIA - SAN SEBASTIÁN (ES); SÁENZ GÓMEZ, Jessica, 20009 DONOSTIA - SAN SEBASTIÁN (ES); OLIVER GARCÍA, Laura, 20009 DONOSTIA - SAN SEBASTIÁN (ES); VIRTO RESANO, Raquel, 31570 SAN ADRIÁN (ES); GONZÁLEZ FERRERO, Carolina, 31570 SAN ADRIÁN (ES); RODRÍGUEZ GONZÁLEZ, María de la O, 31570 SAN ADRIÁN (ES); GARRIDO MARTÍNEZ, María, 31570 SAN ADRIÁN (ES); GARCÍA BARRADO, José Antonio, 06195 VILLAFRANCO DEL GUADIANA (ES)
(74) Representative: ZBM Patents - Zea, Barlocci & Markvardsen

(57) **Abstract**

The invention relates to an isolated strain of *Sporobolomyces roseus* with high carotenoid production capacity. There are also disclosed processes for obtaining colored compositions comprising carotenoids and in particular β-carotene. The invention also discloses the use of these compositions as antioxidant agents and as colored ingredients in edible products. It also relates to processes for reutilizing food industry waste materials or byproducts, such as citrus fruit molasses with *Sporobolomyces roseus* strains.

## Description

The present invention relates to the fields of microbiology, food technology and to processes for obtaining colorants from industrial food waste materials, and particularly, to a novel yeast strain of *Sporobolomyces roseus* that can be used in industrial processes for the production of colored compositions and antioxidant ingredients.

### BACKGROUND ART

Colorants are widely used in food industry in order to give an appropriate color to final products, which are recognized by consumers as food in good conditions.

Production of colorants and in particular of carotenoid pigments by chemical synthesis implies high costs linked to low production yields.

With the aim of improving colorant and in particular carotenoid production, some of them are industrially produced using bacteria, yeasts, fungi, and plants. These colorants produced by means of living organisms are also termed "biocolorants". In the particular case of yeasts, carotenoid production is associated to the response to stress conditions. Carotenoids are organic pigments produced by all these organisms from fats and other basic organic metabolic building blocks. Carotenoids are splitted into two classes, xanthophylls (which contain oxygen) and carotenes (which are purely hydrocarbons, and contain no oxygen). All carotenoids are tetraterpenoids, meaning that they are produced from 8 isoprene molecules and contain 40 carbon atoms. According to their chemical structure and synthesis, carotenoids are tetraterpenoids (40 carbon atoms) derived from the condensation of four terpene units (each unit having 10 carbon atoms). Among carotenoids, the β-carotenes, α-carotene and the β-cryptoxanthin have vitamin A activity in humans. They can also act as antioxidants. β-carotenes is a strongly colored red-orange pigment. β-carotene is biosynthesized from geranylgeranyl pyrophosphate, and comprises beta-rings at both ends of the molecule. Gut absorption of β-carotene is enhanced if eaten together with fats, as carotenes are fat soluble molecules.

Taking profit of the fact that some microorganisms may produce carotenoids, it is widely extended their use for re-using waste materials that are processed by these microorganisms as carbon, nitrogen and energy source. An example of this is the production of colorants from yeast as disclosed in Marova et al. "Use of several waste substrates for carotenoid-rich biomass yeast production", Journal of Environmental Management- 2012, vol. no. 95, pp.:s338-s342. Marova et al. disclose the influence of several types of waste substrates and of stress factors on the production of carotenoids by yeast strains *Rhodotorula glutinis, Rhodotorula mucilaginosa* and *Sporobolomyces roseus.* Some of the waste materials used include potato and whey waste substrate from dairy industry.

An overview of the biotechnological production of carotenoids by yeast is derivable from the document of Mata-Gómez et al. "Biotechnological production of carotenoids by yeast: an overview", Microbial Cell Factories-2014, vol. no. 13:12. The authors disclose some of the conditions needed by yeasts for producing carotenoids, as well as those factors influencing such production (carbon source, solvents, growth medium, etc.). In addition, Mata-Gómez et al., enunciate some of the industrial by-products that are being used as low-cost substrates including grape juice, grape must, sugar cane juice, hydrolysed mustard waste isolates, corn syrup, and milk whey. Table 1 of the document lists some yeast species that have been used for producing carotenoids from agro-industrial wastes as well as which wastes are the substrates for the yeasts. Among these yeasts are mentioned the above-referred yeasts *Rhodotorula glutinis and Rhodotorula mucilaginosa* disclosed by Marova et al *(supra)*

Other carotenogenic yeasts are also used as food ingredients, as the one disclosed in WO2011130576, wherein oleaginous yeast biomass from *Rhodotorula mucilaginosa* DSM 70398 and other yeast strains are used as ingredients in food in the form of yeast oil or whole oleaginous yeast cells. They are mainly used as satiety-inducing agents, antioxidants and/or preservative agents.

Genetically modified and mutated strains of yeasts are also disclosed in the patent literature for the production of compositions comprising β-carotene. Examples include the patent application US20120142082, wherein it is disclosed that some genetically modified yeast *Yarrowia lipolytica* strains are able to produce carotenoids. Also the abstract of the patent JPH0622748 relates to the production of β-carotenes stable in quality and productivity using *Rhodotorula glutinis* selected strains created by mutagenic treatment.

Although there are some microorganisms, along with yeasts, for producing carotenoids, there is still a need of new strains and new industrial conditions allowing high carotenoid production yields for producing biocolorants and antioxidant ingredients, while saving costs and time.

### SUMMARY OF THE INVENTION

The inventors have isolated a new pigment producing strain of *Sporobolomyces roseus* deposited at the Colección Española de Cultivos Tipo (CECT) on 25 March 2015 with the identification reference LGO1107.

The strain of *Sporobolomyces roseus* was isolated from an industrial surface on a food factory from Spain. It was deposited, according to the Budapest Treaty, the 25^{th} of March 2015 in the Colección Española de Cultivos Tipo (CECT) in the Universidad de Valencia C.P 46980 Catedrático Agustín Escardino N° 9 Paterna, Valencia (Spain), by the depositors: 1)CENTRO NACIONAL DE TECNOLOGÍA Y SEGURIDAD ALIMENTARIA (CNTA) (Ctra. Na - 134, Km 50, 31570 SAN ADRIÁN (Navarra), Spain); 2) FUNDACION TECNALIA RESEARCH & INNOVATION (Parque Tecnológico de Miramón, Mikeletegi Pasealekua, 2, 20009 DONOSTIA - SAN SEBASTIÁN, Spain); 3) CENTRO TECNOLÓGICO AGROALIMENTARIO EXTREMADURA (Ctra. Villafranco a Balboa Km 1,2, 06195 VILLAFRANCO DEL GUADIANA (Badajoz), Spain). The strain of *Sporobolomyces roseus* was identified by the depositor with the identification reference LGO1107, and received the CECT accession number CECT 13123 after the International Authority of Deposit declared the strain as viable. It is mentioned indistinctly along this description as *Sporobolomyces roseus* CECT 13123, *S.roseus* CECT 13123, or simply CECT 13123, *S.roseus* LGO1107 or simply LGO1107.

This isolated strain was able to produce β-carotene (and other carotenes and carotenoids) at high yields from citrus waste materials, in particular from citrus fruit molasses, as will be depicted in the examples below. Citrus fruit molasses is a by-product of citrus juice extraction. As a general rule, the fresh pulp obtained after pressing the fruits is mixed with lime and pressed to remove moisture. The resulting liquid (press juice) is filtered to remove larger particles, sterilised by heating and concentrated. The resulting product contains about 72% dry matter and about 64% sugars. Citrus molasses is a thick viscous liquid, dark brown to almost black, with a very bitter taste. It is often sold to distilleries or reincorporated in the dried citrus pulp, but can also be fed directly to animals, or added to grass silage.

Therefore, the yeast strain of the invention provides the advantage of adding value to industrial citrus by-products or sub-products, such as citrus fruit molasses.

The invention also relates to a pure culture yeast which comprises the strain *Sporobolomyces roseus* as defined above. Pure culture means that no other yeast strain or other microorganism is in the culture, but it may optionally comprise compounds of the culture medium wherein cells were grown. Pure cultures are generally provided as lyophilized cultures.

Taking into account the capability of the strain as high yield β-carotenes producer, as well as other carotenoids producer, another aspect of the invention is a process for obtaining a colored composition comprising carotenoids, the process comprising fermenting a culture medium composition comprising a carbon and a nitrogen source with a yeast strain as defined above under aerobic conditions; isolating the yeast strain; and disrupting yeast cell-wall to deliver the composition comprising carotenoids.

Thus, the invention also relates to the use of the strain *Sporobolomyces roseus* CECT 13123 for the production of carotenoids.

Colored compositions obtained by the action of the strain *Sporobolomyces roseus* CECT 13123 imply the advantage of having high concentrations of carotenes, mainly β-carotene, among the synthesised carotenoids. This is so, since the strain is able to produce about 2 µg of carotenes per ml of culture medium composition, quantified by UV-Vis as β-carotenes equivalents at 450 nm. In addition, carotenoid extraction or separation can be performed to obtain colored compositions with higher β-carotene concentrations and/or an isolated carotenoid compound fraction of torularhodin as will be illustrated below. The other main carotenes comprised in the colored compositions obtained by the action of the strain *Sporobolomyces roseus* CECT 13123 include γ-carotene, and torulene.

The invention relates also to a process for obtaining β-carotene comprising carrying out a process as defined above; and extracting the β-carotenes from the colored composition comprising carotenoids.

Besides, the invention encompasses also as an aspect a colored composition comprising carotenoids and obtainable by a process as defined above, said composition comprising β-carotene, γ-carotene, torulene and torularhodin.

These colored compositions may act thus as coloring agents, which means that they might be used to color any substrate wherein they might be comprised, being part of more complex composition (such as food, paint, pharmaceutical composition, etc.).

The colored compositions comprising carotenoids of the invention are particular ones since they result from the delivery of the cytoplasmic cell contents of the yeast. Therefore, they also comprise cellular compounds of the S. *roseus* cell, such as fatty acids. Even in those particular embodiments of the process for obtaining the colored compositions, in which carotenoid extraction or separation steps are performed, the resulting colored compositions comprise some cellular compounds of the S. *roseus* cell that are also extracted or separated together with carotenoids.

Considering well known properties of carotenoid compositions, and in particular of β-carotene, another aspect of the invention is the use of the yeast strain as defined above or of the colored composition as defined above, which comprise carotenoids and in particular high amounts of β-carotenes, as antioxidant agents. This antioxidant activity can be performed, for example, within an oral pharmaceutical or nutraceutical composition, being the colored composition or the yeast by itself either the active principle or the appropriate excipient or carrier for preserving other active substances from oxidation. Antioxidant activity from the yeast itself is provided, for example, when after ingestion by the consumer the contents of the cytoplasm is delivered after digestion.

Indeed, production of carotenoids is achieved by submitting the isolated *Sporobolomyces roseus* CECT 13123 (LGO1107) strain to growth conditions which promote carotenoid synthesis. Therefore, it is another aspect of the invention the use of a strain as defined above, or of a colored composition obtainable as exposed above, as coloring producing agent. A coloring producing agent is to be understood as any compound, composition or cell that provides color to a substrate or matrix where this compound/composition/cell is. Therefore, not only the delivered colored composition embedded in the yeast cells is a coloring producing agent, but also the yeast itself provides said coloring effect, due to its inherent color and to the composition comprising carotenoids embedded in its cytoplasm.

The colored compositions of the invention, as well as the strain of the invention, can be used in food production as colored additives, with the aim of readjusting coloration of the final food product (edible product). It is widely known that color of food actively influences consumer election. In the same way, these colored compositions may be employed in pharmaceutical or cosmetic compositions, for example for coloring tablets or pills, and even liquid suspensions or solutions.

The colored compositions, as well as the strain of the invention, may also be used as food ingredients, with the aim of being consumed by consumers and providing them with the associated healthy effects due to their consumption.

Therefore, it is also another aspect of the invention the use of a strain as defined above, or of a colored composition obtainable as exposed above, as an ingredient of an edible product.

Even another aspect of the invention is an edible product which comprises an effective amount of the strain *S.roseus* LGO1107 as defined above, together with appropriate amounts of other edible ingredients.

The invention relates also to a method for the production of carotenoids comprising fermenting a culture medium composition comprising citrus fruit molasses with a yeast strain of *Sporobolomyces roseus* under aerobic conditions.

### BRIEF DESCRIPTION OF THE DRAWINGS

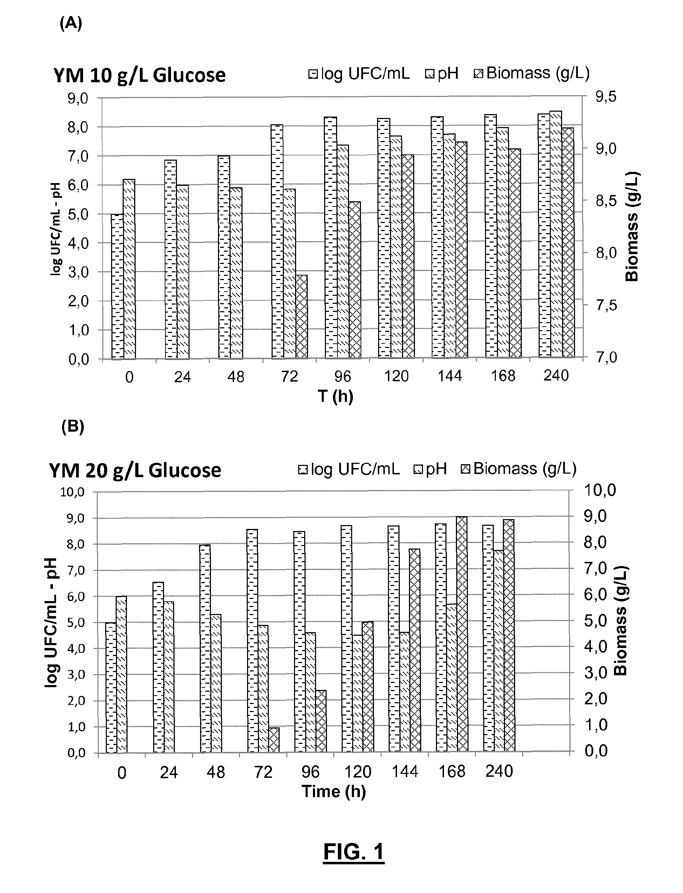

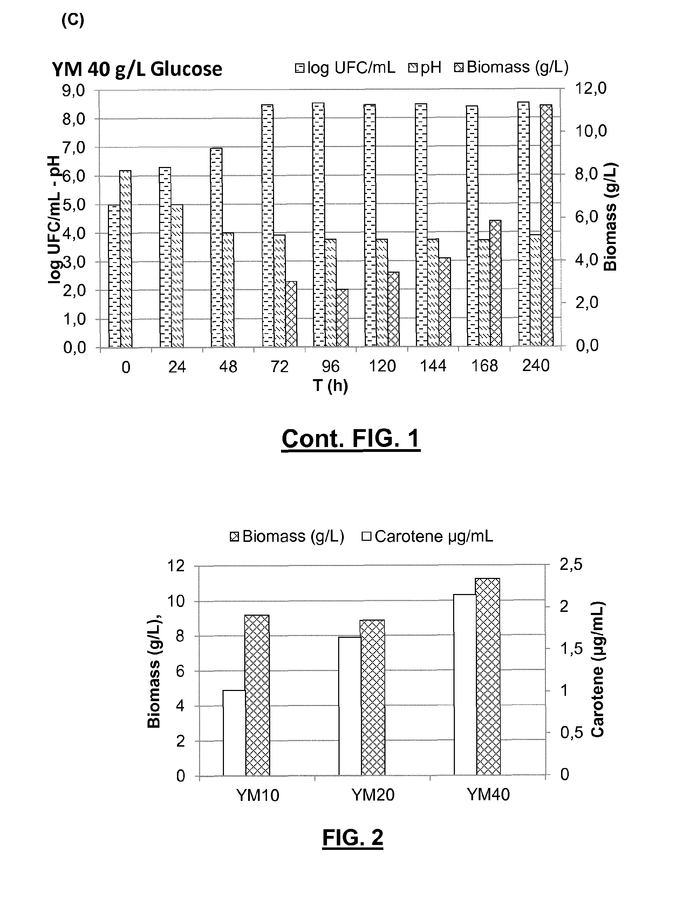

FIG. 1 shows the growing kinetics (log CFU/ml) of *S.roseus* CECT 13123 (LGO1107) in Yeast Malt medium (YM) at different glucose concentrations; 10 g/l (A), 20 g/l (B), and 40 g/l (C). T(h) means time in hours.
FIG. 2, related to Example 2, shows the yield of *Sporobolomyces roseus* CECT 13123 in biomass (g/L), determined by gravimetric measurement after centrifugation and drying and production of carotenes (µg/ml) at 240 hours in Yeast Malt medium (YM) at different glucose concentrations; 10 g/l (YM10), 20 g/l (YM20), and 40 g/l (YM40).

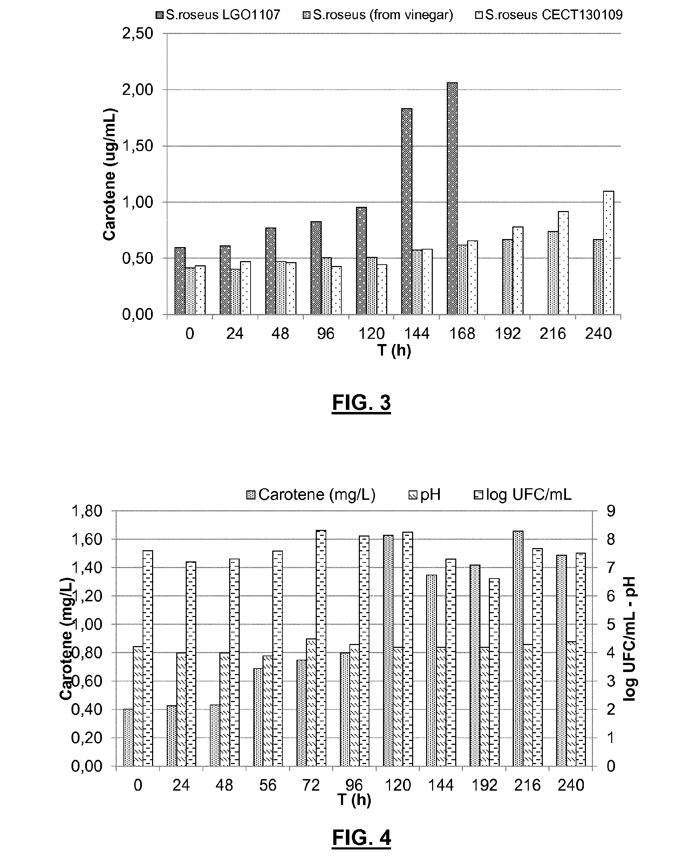

FIG. 3, related to Example 3, is a bar diagram with the compared carotene production (µg/ml of molasses) between *S.roseus* CECT 13123 (depositor's reference: LGO1107) and the reference strain CECT 13019 in mandarin molasses at pH 6. T (h) means time in hours. Data from another vinegar isolated S. *roseus* are also depicted. As above T(h) means time in hours.

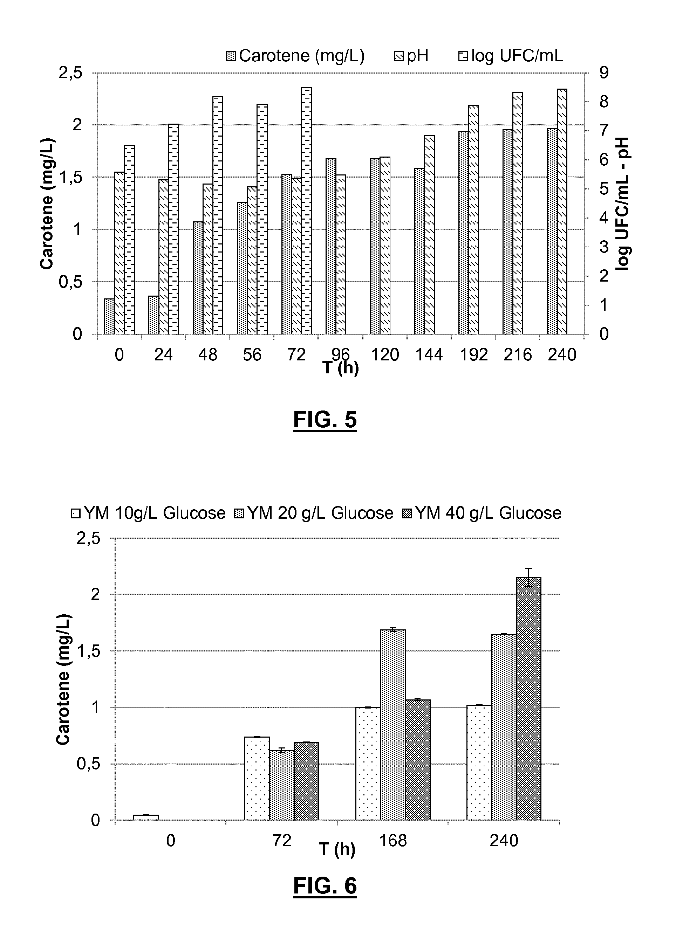

FIGs. 4 and 5, show respectively the growing kinetics (log CFU/ml) of *S.roseus* CECT 13123 in mandarin molasses in a volume of 3 litres or 30 litres. T (hour) means time in hours. pH of the medium was also recorded at each assayed time. Production of carotenes (mg/L of media) is also recorded. As above T(h) means time in hours.

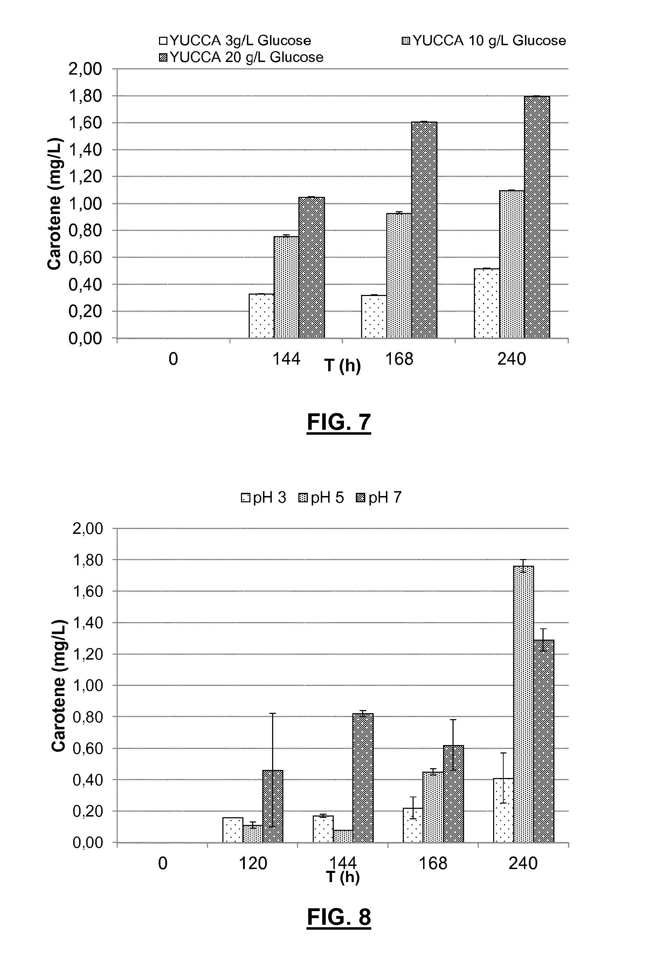

FIGs. 6 and 7 relating to Example 4 show, respectively, the carotene production (mg/L) of *S.roseus* CECT 13123 in differently supplemented YM and YUCCA media in order to see the influence of glucose in fermentation parameters. As above T(h) means time in hours.
FIG. 8, also relating to Example 4, shows the effect of the pH of the culture media in the production of carotenes. As above T(h) means time in hours.

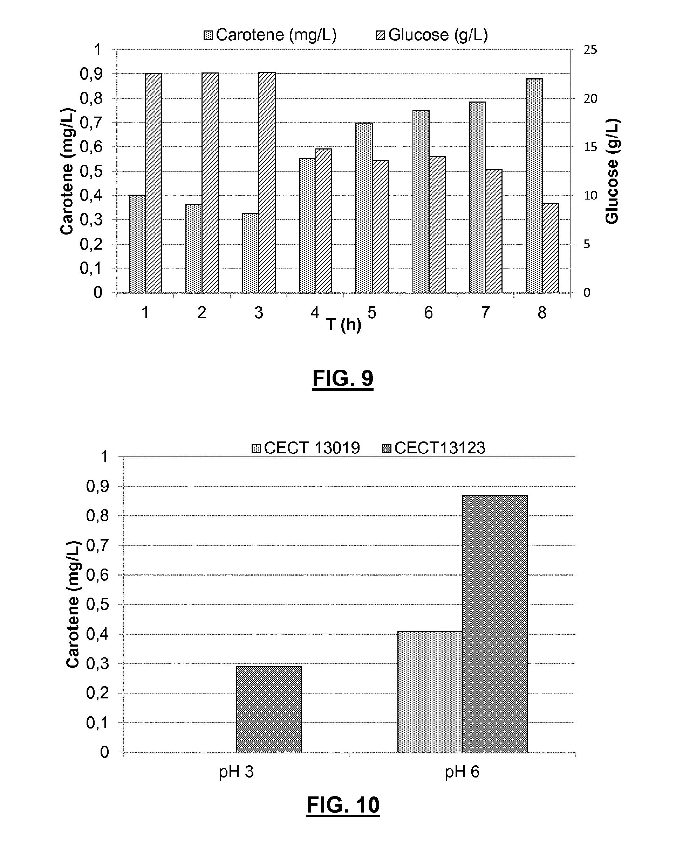

FIG. 9, relating to Example 5, is a bar diagram showing the glucose levels (g/l) and the produced carotenes (mg/L) amounts along time (T in hours; T(h)) when culturing strain *S.roseus* CECT 13123 (LGO1107) in orange molasses.
FIG. 10, relating to Example 6, is another bar diagram showing the effect of pH of the medium in the amount of β-carotenes (µg/ml) produced by S. *roseus* LGO1107 and by the reference S. *roseus* CECT 13019. The culture medium composition comprises mandarin molasses, and the assayed pH are pH 3 and pH 6.

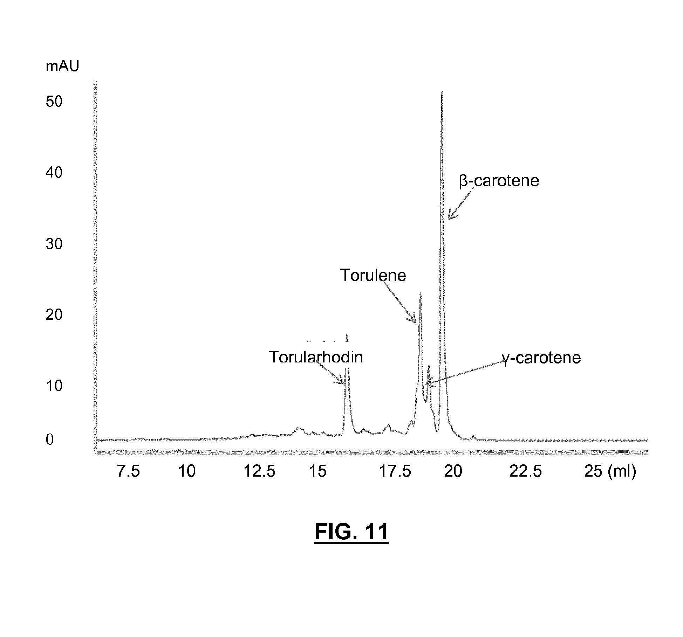

FIG. 11, relating to Example 9, is chromatogram of carotenoids extracted from *Sporobolomyces roseus* CECT 13123. Detection and quantification method by HPLC-DAD. mAU are mili absorbance units (Y-axis); elution volume in ml (X-axis). Peaks corresponding to tolurene, β-carotene, γ-carotene and tolurahodin are depicted for a sample extracted from fermentation of PDA medium by CECT 13123.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition. The following definitions are included for the purpose of understanding.

For "food ingredient" is to be understood any substance that either becomes part of a food product, either directly or indirectly, during some phase of its processing, storage or packaging or can be consumed alone as food. Included in this definition, for "food additive" is to be understood any substance added to a food for a specific purpose in that food: the intended use of which results - - directly or indirectly -- in its becoming a component of the food or otherwise affecting the characteristics of any food. The strain of the invention or the colored composition obtainable from it may be used or conceived independently as food ingredient as a whole or even specifically as food additive. As a way of example, if the composition comprising carotenoids (i.e. β-carotene) is in a final food to be consumed and with the aim of producing a particular effect on the organisms (promote synthesis of A vitamin, for example), then it is conceived as an ingredient. If, on the other side, the composition is added to the food in order to preserve it until its expiration date, it is conceived as an additive. Concentration in the final food will be adjusted accordingly depending on the desired function.

For "color additive" is to be understood any substance or composition that can be added to something else to cause a change of color. A color additive (also termed herewith coloring agent or coloring producing agent) is any dye, pigment, substance or composition which when added or applied to a food, drug or cosmetic, or to the human body, is capable (alone or through reactions with other substances) of imparting color. A "colored composition comprising carotenoids" is thus to be understood as a composition with the ability to change the color of any substrate (other composition for example), where it is added.

For "antioxidant agent" is to be understood a molecule or composition inhibiting oxidation of other molecules. Oxidation is a chemical reaction involving the loss of electrons or an increase in oxidation state. Oxidation reactions can produce free radicals. In turn, these radicals can start chain reactions. When the chain reaction occurs in a cell, it can cause damage or death to the cell. Antioxidants and in particular carotenoids terminate these chain reactions by removing free radical intermediates, and inhibit other oxidation reactions. They do this by being oxidized themselves, so antioxidants are often reducing agents able to quench reactive oxygen species (ROS).

For "edible product" is to be understood any food that is orally taken by consumers. Examples of foods include fruit juices, refreshing drinks, shakes, dairy products (milk, yogurt, and butter), vegetable creams, and confectionery among others. The "effective amount of the colored composition or of the yeast strain" relates to a non-toxic amount of this composition producing, in the composition wherein it is added, a color change besides acting as antioxidant agent or as simply edible ingredient. An "edible ingredient" is any compound of an edible product that can be consumed orally.

The "oral pharmaceutical compositions" or "nutraceutical compositions" comprising the effective amounts of any of the colored composition or of the yeast strain include tablets, pills, capsules, and liquid suspensions or solutions. The strain or the colored compositions are added in this kind of compositions in an effective non-toxic amount, able to produce the desired effect without being dangerous for the consumer. These pharmaceutical compositions are accompanied (or comprise) pharmaceutically excipients and/or carriers. Nutraceutical compositions are concentrated nutrients and/or vitamin and/or mineral sources taken by animals or humans in order to complete their feeding. Cosmetic compositions wherein effective amounts of any of the colored composition or of the yeast strain are included, comprise gels, creams, ointments, serums, powders, mousses, lotions, sticks, ointments, pastes, shampoos, shower gel, body washes or face washes, wherein any acceptable cosmetically excipients and/or carriers are accompanying the colored composition of the invention or the strain CECT 13123.These cosmetic compositions take benefit of the effective amounts of any of the colored composition or of the yeast strain in particular due to their antioxidant activity.

For "β-carotene equivalents" is to be understood the total amount of carotenes of any type in a solution that give the same absorbance units (A.U.) that will correspond to a solution with pure β-carotenes (with no other carotene type compound).

The invention provides an isolated strain of *Sporobolomyces roseus* with worth advantages over other strains of *Sporobolomyces roseus,* since it is a high yield carotenoid producer under aerobic conditions and in particular a high β-carotene producer, which is a widely used pigment in many industries, including food industry. In addition, this strain is able to grow in agro-industrial waste material containing substrates, such as in citrus by-products or sub-products, including citrus fruit molasses. Therein, the strain is also able to manufacture carotenoids from these sub-products, in particular carotenes (mainly β-carotene), adding value to them while reducing the impact of waste material.

The strain *of Sporobolomyces roseus* CECT 13123 (LGO1107) is used for producing carotenoids. The strain CECT 13123 can be used also in a process for obtaining colored compositions comprising said carotenoids (among which β-carotenes is present), said process comprising fermenting a culture medium composition that provides the elemental nutrients for growing the yeast strain under aerobic conditions. These elemental nutrient ingredients are a carbon source, such as carbohydrates (to obtain energy and building blocks for cell walls) and a nitrogen source, such as amino acids, basically used for the synthesis of proteins. Since carotenoids are non-secreted compounds that remain inside the yeast cell, disruption of yeast cell-wall or extraction in any mode from the interior of the cell is compulsory in order to deliver the carotenoids (colored composition comprising carotenoids).

In a particular embodiment, the process to obtain colored compositions is characterized by comprising the steps of:
(a) preparing a pre-culture of the strain of *Sporobolomyces roseus* CECT 13123 as defined above;
(b) inoculating a culture medium composition comprising a carbon and a nitrogen source with the pre-culture of step (a);
(c) aerobically fermenting the carbon source of the culture medium composition;
(d) isolating the yeast strain; and
(e) disrupting yeast cell-wall to deliver the composition comprising carotenoids.

It is easy to note that the above-referred method can be stopped in (d) if desired. Then, a method for isolating the strain of the invention with the colored composition constituting the cytoplasm of the cell is performed. As will be indicated below, these isolated cells in entire form are, as such, coloring producing agents, due to its inherent coloring.

In yet a more particular embodiment, the pre-culture is a preliminary small-scale culture of the strain used to inoculate the culture medium composition wherein fermentation will be carried out. In another particular embodiment, the pre-culture of the strain of the invention can be carried out, , in a yeast culture media with a modified pH between 3 and 6. This pre-culture aims to prepare the strain to grow effectively in the fermentation media, wherein the production of the carotenoids will take place. An example of this yeast culture media for preparing the pre-culture is the YUCCA medium comprising 10 g/L glucose, 3 g/L yeast extract,2 g/L KH2PO4, 0,5 g/L MgSO4 (pH 6), where the strain is grown under the aerobic culture conditions explained below.

Also in another more particular embodiment, optionally in combination with any embodiment above or below, the culture medium composition of step (b), comprising the carbon and nitrogen source, further comprises or consists of citrus fruit molasses. Examples of molasses include those of mandarin, orange, lemon, grapefruit, lime, and mixtures thereof. More particularly, the citrus fruit molasses is mandarin molasses or orange molasses.

These culture medium composition comprising a carbon and a nitrogen source (being molasses or not) refers to a culture medium containing digestible energy, carbon and nitrogen used by the yeast strain. For example, among suitable carbon and energy sources are found glucose, xilose, fructose, sucrose, lactose, galactose, and mixtures thereof. As suitable nitrogen sources can be included, but without being limited to them, yeast extract, meat extract, corn steep liquor (CSL), meat, soybean or casein peptones, protein hydrolysates from protein-rich agro-food by-products, such as soybean meal/flour, whey, dry distillers grains with solubles (DDGS), and mixtures thereof.

As exposed above, these molasses are obtained as by-products of citrus juice extraction. When citrus fruit molasses are employed as culture medium, they are previously treated to reduce the solid content. This treatment may include steps such as precipitation and/or filtering and/or decantation of solid particles of the citrus fruit performed with the aim of separating those solids that will not be usedin the fermentation. In a particular embodiment, the citrus fruit molasses are clarified until a solid residue is in the range from 1% to 10% by weight, in particular from 2.5% to 5% by weight.

The invention provides also a process for the production of carotenoids comprising fermenting a culture medium composition comprising citrus fruit molasses with a yeast strain of *Sporobolomyces roseus* under aerobic conditions; and disrupting the yeast cell-wall to recover carotenoids. As exposed above, a particular embodiment of this process is the one carried out with strain LGO1107 using as culture medium a composition comprising or consisting of citrus fruit molasses.

In order to deliver carotenoids from inside of yeast cells, the cell-wall has to be disrupted, understanding as disruption both the formation of pores in the yeast cell-wall or cell membrane, or the formation of any other impairment on the yeast cell-wall or membrane allowing the release of the cytoplasmic contents. In a particular embodiment, disruption is performed by centrifugation, by mill procedures, or with high voltage electric pulses, combined with organic solvent use, which means in an organic solvent.

In a particular embodiment of the process, disruption of yeast cell-wall is carried out in a liquid media comprising the polar aprotic solvent dimethyl sulfoxide (abbrv. DMSO), which can optionally be pre-heated from 40 °C to 50 °C. Sonication and vortexing are also additional suitable techniques for disrupting yeast cell-wall. The liquid media can be the solvent itself or a known yeast culturing media, such as the YUCCA media or the YM media, comprising said apolar organic solvent.

In another particular embodiment, optionally in combination with any embodiments above or below, disruption of yeast cell-wall is performed by mill procedures in an organic solvent selected from diethyl ether, ethyl acetate, acetone, hexane, ethanol, petroleum ether, and combinations thereof. Yet in another particular embodiment, disruption is performed with high voltage electric pulses in any of these organic solvents.

Linking with the techniques or additional steps for recovering compositions comprising carotenoids, in another more particular embodiment, the process comprises additional steps in order to achieve the isolation of specific carotene compounds, such as purified β-carotene. "Purified β-carotene" is to be understood in the sense of the present invention, as a composition that is recovered by means of any of the above or below mentioned technologies, and wherein the percentage by weight (in g/g) of β-carotenes in relation to the total amount of isolated carotenes is of at least 90 %.

In a particular embodiment of the method for obtaining β-carotene, or for obtaining colored compositions with carotenoids (mainly carotenes), the method comprises additional liquid extraction steps, or extraction steps in combination with distillation, or other techniques which the skilled man will know and might be used for the isolation of the different carotenes.

Having in mind the particular chemical and physical properties of carotenoids, fractions enriched with particular carotenoids can be obtained by applying several distillation series, or alternatively by several solvent extraction (solid phase or liquid-liquid extraction) steps, or alternatively by using chromatographic techniques the skilled man will know. Examples are provided below.

Thus, in another particular embodiment of the process, after disrupting the yeast cell-wall, an additional step for recovering carotenoids is performed. In particular, the recovery is performed by means of an extraction step in an apolar organic solvent (non-polar organic solvent). In a particular embodiment, the organic solvent is an apolar organic solvent selected from diethyl ether, ethyl acetate, acetone, hexane, ethanol, petroleum ether, and combinations thereof. Using the organic solvent allows recovering the carotenoids or the colored composition produced by the yeast strain from other compounds of the cytoplasm of the yeast cell. Depending on the solvent used, it could also be used with the aim of separating (fractionating) individual carotenoid compounds produced by the strain.

In a particular embodiment, the solution comprising carotenoids is dried to obtain the carotenoids in dried form, or alternatively the solution comprising carotenoids is submitted to the precipitation of the carotenoids, that are later recovered from the precipitate.

Any of the forms in which the colored composition comprising carotenoids is provided can be used as coloring producing agent or as antioxidant agent within more complex compositions (food, oral pharmaceutical or nutraceutical compositions).

In a more particular embodiment, obtaining of or delivering of the colored composition comprising carotenoids is performed by:
- isolating yeast cells from the fermented culture medium of step (c) by centrifugation means;
- re-suspending and disrupting yeast cells in an organic solvent, particularly dimethyl sulfoxide (DMSO), and wherein the solvent is in particular pre-heated at a temperature ranging from 40 to 50 °C;
- extracting the re-suspended and disrupted yeast cells with an organic solvent selected from diethyl ether, ethyl acetate, acetone, hexane, ethanol, petroleum ether, and combinations thereof, particularly the combination diethyl ether/ethyl acetate (1:1); and
- centrifuging the mixture to obtain two phases, and
- recovering the upper organic phase consisting in the colored composition comprising carotenoids.

In any of the processes for obtaining colored compositions comprising carotenoids, or for obtaining β-carotene, step (b) is carried out with a strain inoculum concentration from 10³ to 10⁸ colony formation units per ml of culture (CFU/ml), in particular from 10⁴ to 10⁷ CFU/ml. In a more particular embodiment, it is from 10⁵ to 10⁶ CFU/ml of culture medium. In a more particular embodiment the total volume of culture medium composition is from 15 to 60 litres (L). From 10³ to 10⁸ includes 10³, 10⁴, 10⁵, 10⁶, 10⁷ and 10⁸ CFU/ml.

In another particular embodiment of the process of the invention, optionally in combination with any embodiment above or below, the step of fermenting the culture medium is performed in a temperature range from 20 °C to 35 °C, more particularly from 20 °C to 30 °C, and even more particularly from 20 °C to 25 °C. From 20 °C to 35 °C means any temperature selected from 20, 21, 22, 23, 24, 25, 26, 27, 28, 29, 30, 31, 32, 33, 34 and 35 °C.

In another particular embodiment, optionally in combination with any embodiment above or below, the step of fermenting the culture medium is carried out in a pH range from 2.0 to 8.0, more particularly from 3.0 to 6.0. Thus, pH is selected from 2.0, 3.0, 4.0, 5.0, 6.0, 7.0, and 8.0.

When aerobic conditions are mentioned in the present invention, they comprise in particular oxygenation embodiments (provision of oxygen source) at a rate flow of 2 to 10 L/min, particularly of 5 L/min, from 20 %v/v-30 % v/v, wherein v/v means volume of air containing until 21 % of O₂ per volume of culture medium, and constant agitation of the culture medium composition, thus aiding an homogenous oxygenation of the whole volume.

In another particular embodiment of the processes, the step of fermenting is carried out for a period ranging between 7 to 12 days, more particularly for 10 days.

By means of all these embodiments of the processes, colored compositions comprising carotenoids, particularly comprising β-carotene, may be obtained. Thus, in a particular embodiment the colored compositions are obtainable by a process comprising fermenting a culture medium composition comprising a carbon and a nitrogen source with the strain of *Sporobolomyces roseus* CECT 13123, or with a pure culture of it under aerobic conditions; and disrupting yeast cell-wall to deliver the composition comprising carotenoids.

More particularly, the colored compositions are obtainable by:
(a) preparing a pre-culture of the strain of *Sporobolomyces roseus* CECT 13123, or a pure culture of it;
(b) inoculating a culture medium composition comprising a carbon and a nitrogen source with the pre-culture of step (a), comprising said culture medium composition citrus fruit molasses or even consisting only of these citrus fruit molasses;
(c) aerobically fermenting the carbon source of the culture medium composition;
(d) isolating the yeast strain, and
(e) disrupting the yeast cell-wall to deliver the colored composition comprising carotenoids from the inner of the cells.

The colored compositions so obtained comprise β-carotenes, among other carotenoids (including xanthophylls and other carotenes). The colored compositions so obtained comprise in particular β-carotene, γ-carotene, torulene and torularhodin. In a particular embodiment of the colored compositions of the invention the percentage by weight of β-carotene is at least 40 % in relation to the total of carotenes in the composition (w/w in grams). The percentage by weight indicated herewith is calculated by recovering carotenes of the colored composition by means of disrupting yeast cells with DMSO pre-heated at 50 °C, and extracting the disrupted yeast cell with diethyl ether/ethyl acetate (1:1). This is the reference analytical method proposed by the inventors for comparing the results obtained with other yeast strains.

In another particular embodiment, the colored composition comprising carotenoids comprises β-carotenes in a percentage by weight ranging from 48 to 50 %; γ-carotene in a percentage by weight ranging from 10 to 12 %; torulene in a percentage by weight ranging from 26 to 28 %; and torularhodin in a percentage by weight ranging from 12 to 14 %, all percentages by weight in relation to the total amount of carotenes which amount to 100 %. As indicated above, the colored composition comprising carotenoids also comprises other carotenoids, in particular xanthophylls. These weight percentages of carotenes in the colored composition are determined, as indicated above, by recovering carotenes with the procedure comprising DMSO disruption and diethyl ether/ethyl acetate extraction.

Considering the known antioxidant properties of β-carotenes, the colored compositions of the invention are proposed for their use as antioxidant agents, as well as coloring producing agents within complex compositions. Examples of complex compositions include edible compositions (food), nutraceutical and pharmaceutical compositions or cosmetic compositions.

Non limitative examples of products wherein the colored compositions of the invention may be added include pharmaceutical compositions (in particular oral pharmaceutical compositions), nutraceutical compositions, cosmetical compositions, and food, in particular fruit juices, refreshing drinks, shakes, dairy products (milk, yogurt, butter), vegetable creams, and confectionery among others.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Example 1. Isolation of strain Sporobolomyces roseus CECT 13123 (LGO1107)

The strain of *Sporobolomyces roseus* of the invention was isolated from a surface in a food industry and according to the following procedure:
From surface Rodac plates with sabouraud chloramphenicol agar incubated during five days at 25°C, five bacterial colonies were selected and isolated looking after their red color. Several incubations were made in Potato Dextrose Aagar (PDA) before their analysis.

The identification was performed using the restriction fragment length polymorphism (RFLP) technique of the internal transcribed spacer (ITS) and sequencing the rRNA appropriate region to know the specific strain with the Basic Local Alignment Search Tool (BLAST).

**Table 1. Molecular weight by RFLP-ITS**

| Sample Internal Code | Genre and species | ITS | Hhal | Haell | Hinfl |
|---|---|---|---|---|---|
| LGO1107 | *Sporobolomyces sp* | 610 | 300+200+80 | 600 | 280+120+95+95 |

**Table 2.sequencing result**

| Sample Internal Code | RFLP Profile | % BLAST homology |
|---|---|---|
| LGO1107 | *Sporobolomyces sp* | 99.9% *roseus* |

All these data confirmed that the isolated strain was an *S.roseus* strain.

As above indicated the isolated LGO1107 strain was deposited at Colección Española de Cultivos Tipo (CECT) and received the accession number CECT 13123.

### Example 2. Growing and carotene production of Sporobolomyces roseus CECT 13123 (LGO1107) in YM broth

In order to better characterize the new isolated strain, its growth was analysed in a Yeast Malt (YM) broth formulated with addition of different amounts of glucose (principal sugar in the molasses by-product): 10.00, 20.00 and 40.00 g/L of glucose. In these conditions, the carotene production was also evaluated.

Composition of YM broth was: Peptic digest of animal tissue (5.00 g/L), Yeast extract (3.00 g/L), Malt extract (3.00 g/L), Dextrose (10.00 g/L)

Growing kinetics and carotene production are depicted in FIG. 1 (A to C). Production of carotenes was determined as exposed in Example 8, with the procedure comprising DMSO disruption and Diethyl ether/Ethyl Acetate (1:1) extraction and quantification with spectrophotometry assay UV-Vis at 450 nm.

FIG. 2 shows the yield of *Sporobolomyces roseus* CECT13123 in biomass (g/L), determined by gravimetric measurement after centrifugation and drying and the carotene production at 240 hours.

Conclusion from these data is that *Sporobolomyces roseus* CECT13123 produces high amounts of carotenes in this culture media.

### Example 3. Colored compositions from fermentation of mandarin molasses with yeast strains. Comparison of Sporobolomyces roseus CECT 13123 with other Sporobolomyces roseus strains

*Sporobolomyces roseus* CECT 13123 (LGO1107), the reference strain *Sporobolomyces roseus* CECT 13019 (commercially available) and another isolated from vinegar *Sporobolomyces roseus* strain were cultured and assayed to obtain colored compositions comprising carotenoids.

The strains (previously maintained at 4 °C in potato dextrose agar) were inoculated in a synthetic YUCCA broth (culture medium composition) comprising yeast extract (2g/L) 10 g/L glucose, 10 g/L KH2PO4, 2g/L MgSO4 (pH 6.). They were cultured for 24 h at 23 °C with constant oxygenation and agitation.

In a lab scale, 10 ⁵⁻⁶ CFU/ml of each strain from the pre-cultures were inoculated in 3 L of mandarin molasses. Fermentation was carried out for 10 days (240 h) with light conditions and at a temperature of 23 °C. Agitation was of 200 rpm (Biostat ® B Plus) and oxygenation maintained constant with a flow of 25 % v/v by means of Biostat ® B Plus.

Mandarin molasses was obtained from a citrus fruit derivatives provider, and its composition was as follows: humidity (95 g/100 g), nitrogen (0.32/100 g), ashes (0.17g/100g), carbohydrates (4.5 g/100 g), fructose (0.10 g/100g), glucose (3.9 g/100g), sacarose (0.32 g/100g) and pH 3.4. Molasses was clarified by filtering and pH was adjusted to 6 before inoculation of the strains.

In FIG. 3 the total carotene production between the three different strains of *S.roseus* in mandarin molasses at pH 6 in 3 L fermentator is depicted in bars. Carotene amounts were determined as in Example 8, with the procedure comprising DMSO disruption and Diethyl ether/Ethyl Acetate (1:1) extraction, and quantification by spectrophotometry assay UV-Vis at 450 nm, as β-carotene equivalents.

*Sporobolomyces roseus* CECT 13123 (LGO1107) appears as the best carotene production in modified pH mandarin molasses, even better that the reference CECT 13019. The results of FIG. 3 show that *S.roseus* LGO1107 produced a higher amount of colored composition comprising carotenoids, in particular carotenes, from citrus fruit molasses than the reference CECT 13019. In addition, LGO1107 production was faster than CECT 13019 production, since at each of the assayed times a higher production was observed.

With the aim of further characterizing the growing kinetics and behaviour of *Sporobolomyces roseus* CECT 13123, several pH adjustments were done in mandarin molasses were CECT 13123 was inoculated as above indicated. Log of CFU/ml and the carotene production (mg/L) were recorded. Data are depicted in FIG. 4, which shows the growing kinetic (log CFU/ml) versus time in hours of *S.roseus* CECT 13123 in mandarin molasses in a volume of 3 litres. pH of the medium at each time is also depicted.

In a pilot scale, *Sporobolomyces roseus* CECT 13123 (LGO1107) was also inoculated at the same initial concentration than in lab scale but in a final volume of the mandarin molasses of 30 L. Fermentation was conducted for 10 days at 23 °C, 680 rpm (Reactor Biológico RB-50, Energesa S.A.) and constant oxygen supply. Results are depicted in FIG. 5, wherein the growing kinetics (log CFU/ml) versus time in hours of *S.roseus* CECT 13123. Stationary phase was reached at 72 h and growing data aren't depicted from 96 h to 240 h.

Both FIGs. 4 and 5 demonstrate that the new strain was able to grow at high yields in mandarin molasses and also able to produce carotenes at high concentrations.

The strain of *Sporobolomyces roseus* CECT 13123 (LGO1107) was thus also useful to accomplish fermentation at industrial levels. Without being bound to theory, inventors think that this is partially due to the fact that this strain has a high growing rate in citrus fruit molasses in relation to other strains of *Sporobolomyces roseus.*

### Example 4: Influence of the fermentation conditions on the carotene production by Sporobolomyces roseus CECT 13123

Fermentation conditions for optimization of the carotene production were studied.

It was analysed the carotene production in YM and YUCCA media with different glucose concentrations. Culture conditions and inoculation were the same as indicated in Example 3 (only the culture media were changed). Data are depicted in FIG. 6, for supplemented YM; and in FIG. 7, for supplemented YUCCA medium. The higher the glucose content, the higher the carotene production (in mg/L) considering the whole culture time (240 hours).

pH effect of the growing media can be seen in FIG. 8. The growth of CECT 13123 in YUCCA supplemented with 20 g/L glucose was assayed at several pHs (pH 3, 5 and 7). At several growing times carotene production (mainly of β-carotene among other carotenes) was determined as exposed above (as in Example 8, with the procedure comprising DMSO disruption and Diethyl ether/Ethyl Acetate (1:1) extraction and quantification with spectrophotometry assay UV-Vis at 450 nm). Surprisingly, even at low pH (those of citrus fruit molasses) production of carotenes was achieved. Best production in YUCCA was obtained at pH 5-7.

### Example 5. Growing of Sporobolomyces roseus CECT 13123 (LGO1107) in orange molasses

With the aim of determining if LGO1107 strain was able to grow and to produce carotenoids, growing was performed in orange molasses at pH 5,5 Recovery of carotenes and quantification as indicated in Example 8, with the procedure comprising DMSO disruption, Diethyl ether/Ethyl Acetate (1:1) extraction and quantification with spectrophotometry assay UV-Vis at 450 nm as β-carotene equivalents.

LGO1107 was able to grow also in orange molasses. Carotene production (mainly β-carotene, among other carotenes) is depicted in FIG. 9. In this figure, the first bars are the carotene amounts (mg/L media) produced at each sampled time (in hours). Second bars correspond to the glucose levels (g/L media) in the growing media at the same samples time.

This example shows the capability of the strain of the invention for fermenting another citrus fruit material (orange molasses), which makes it a versatile strain useful in several citrus fruit industries waste materials.

### Example 6. Effect of pH of culture medium composition comprising mandarin molasses

*Sporobolomyces roseus* CECT13123 (LGO1107) and CECT 13019 were grown in mandarin molasses as the ones indicated in Example 3.

Culturing was done in an Erlenmeyer (0,3L medium volume) for 240 hours. The cell growing and pH was monitored daily during the fermentation time in order to control de fermentation process.

pH of the media was adjusted at 3 or 6. As can be seen in FIG. 10, the strain of the invention (LGO1107) was able to produce carotenes even in acidic conditions (pH 3), were it was able to grow. Not so resulted from reference strain CECT 13019. As exposed above, at pH 6 the strain of the invention produces greater amounts of carotene than the reference strain. (Recovery of carotenes and quantification, as β-carotene equivalents, as indicated in Example 8, with the procedure comprising DMSO disruption and Diethyl ether/Ethyl Acetate (1:1) extraction and spectrophotometry assay UV-Vis at 450 nm).

Thus, *Sporobolomyces roseus* CECT 13123 (LGO1107) was able to grow in mandarin molasses with higher yields than the reference CECT 13019, albeit this later was also able to grow in this media and also produced carotenoids., However, *Sporobolomyces roseus* LGO1107 with a high yield of carotenoid production and with a better adaptation to this particular media, supposes an advantageous strain with a real industrial contribution.

These data demonstrate versatility in relation to the culture media of the strain of the invention in comparison with reference strain. Thus, strain of the invention is not only able to grow in several culture media comprising citrus fruit molasses, but also it is able to produce carotenoids in all these tested conditions. Production of carotenoids even under severe stress conditions (for example pH 3) makes strain CECT 13123 (LGO1107) a really interesting strain for food industry, namely in the revalue of waste material from citrus food industry.

### Example 7. Comparison of Sporobolomyces roseus CECT 13123 (LGO1107) with Rhodotorula mucilaginosa DSM 70398

Strain LGO1107 was also compared with *Rhodotorula mucilaginosa,* another yeast known to produce carotenoids (as disclosed in WO2011130576), in a lab assay in Yeast Malt (YM) medium supplemented with glucose at different concentrations (10, 20 and 40 g/l). Inoculums were done at concentration 10⁵ cell/ml and the growing for 240 hours in Erlenmeyer flasks. pH 6 in 3 L fermentator. Data are depicted in next Table 3.

**Table 3. β-Carotene equivalents (µg / g dry biomass) production comparison**

| Broth | *R. mucilaginosa* DSM 70398 | *S. roseus* LGO1107 |
|---|---|---|
| YM + 10 g/L glucose | 6.39 | 10.00 |
| YM + 20 g/L glucose | 11.18 | 18.40 |
| YM + 40 g/L glucose | 13.09 | 25.28 |

| | | |
|---|---|---|
| (quantified by UV-Vis as β-carotene equivalents at 450 nm) (µg / g dry biomass) | | |

From this comparison, it is directly derived the advantage of using *S.roseus* CECT 13123 (LGO1107) as carotenoid producer. At the same culture conditions it was able to produce higher amounts of carotenes (mainly β-carotene) than other yeast known also as a high carotenoid producer.

All the herewith exposed data allow so concluding that an industrial fermentation process could be established adding value to citrus fruit by-products of food industries. This was accomplished with a strain of *Sporobolomyces roseus,* which is a high carotenoid producer in relation to other yeast strains, and that can produce such carotenoids in a medium comprising molasses from agro-industrial waste materials, even in severe stress conditions for yeast, such as a low pH.

### Example 8. Effect of cellular disruption and carotene extraction methods for carotene recovery from the R.glutinis, R.mucilaginosa or S.roseus isolated dried biomass.

The inventors focused also on the provision of a method for standard determination of carotene recovery after fermentation with yeast strains, and also on the provision of a recovery method acceptable in the food industry where the colored compositions of the invention may be used.

Different methods are identified in the bibliography as useful to achieve the yeast cell wall breakage and also a high number of organic solvents and combinations to carotene extraction from the inside of the cell. Next Table 4 summarizes the main carotene recovery methods (in columns the sequential procedure steps).

**Table 4. Carotene recovery methods**

| CELL WALL DISRUPTION | | | |
|---|---|---|---|
| DMSO | DMSO | MILL | HIGH-VOLTAGE ELECTRIC PULSES |
| Sonication | Vortex | | |
| Temperature | Temperature | | |
| | | Organic solvent | Organic solvent |
| | | | |

| CAROTENE EXTRACTION | | | |
|---|---|---|---|
| Centrifugation | | Filtration | |
| Organic solvent | | | |
| Agitation | | | |
| Centrifugation | | | |
| Saline solution | | | |
| Decantation | | | |
| | | | |

| CAROTENE CONCENTRATION | | | |
|---|---|---|---|
| Solvent evaporation | | | |

The inventors want to note that the recovery of carotenoids depends on the specific conditions of the methods of disruption and of carotene extraction (temperature, solvents, time and kind of agitation...) but also on the yeast or bacteria cell strain, which has also a strong influence due to differences on the wall resistance of each strain.

To compare different strains and methods, the results in the following tables are related to the dry biomass used in each breakage and extraction procedure.

Next Tables 5 and 6 are only illustrating examples not-related with the strain of *S.roseus* CECT 13123 of the invention. But they are included to note the importance of considering comparative experimental conditions.

**Table 5. (Reference Example) Solvent comparison of the carotene extraction from R.glutinis grown on YM broth after the use of DMSO (50 °C) and vortex agitation (quantified by UV-Vis as β-carotene equivalents at 450 nm).**

| SOLVENT | CAROTENE CONCENTRATION (µg/g dry pellet) |
|---|---|
| Diethyl Ether | 97.88 ± 8.82 |
| Diethyl Ether / Ethyl Acetate (1:1) | 128.07 ± 6.53 |
| Petroleum Ether | 114.50 ± 9.27 |
| Petroleum Ether / Acetone (1:2) | 120.61 ± 6.03 |

This table illustrates the variability due to the nature of the solvent used in the extraction. Diethyl Ether / Ethyl Acetate (1:1) allows the higher extraction yields.

**Table 6. (Reference Example) Comparison of carotene recovery in DMSO methods from R.mucilaginosa and R.glutinis (quantified by UV-Vis as β-carotene equivalents at 450 nm)**

| METHOD | TIME (hours) | CAROTENE CONCENTRATION (µg/mL) | |
|---|---|---|---|
| | | R. mucilaginosa | R.glutinis |
| DMSO (50 °C)and sonication | 24 | 0.0636 ± 0.0190 | |
| | 48 | 0.212 ± 0.00160 | 0.799 ± 0.016 |
| Hexane/Acetone/Etanol (2:1:1) as organic solvent | 73 | 0.326 ± 0.00620 | 1.035 ± 0.059 |
| | 96 | 0.317 ±0.00190 | 1.499 ± 0.122 |
| | 162 | 0.620 ± 0.0303 | 1.852 ± 0.0550 |
| | 168 | 0.612 ± 0.470 | 1.403 ± 0.204 |
| DMSO (50 °C) and vortex agitation | 24 | 0.190 ± 0.054 | |
| | 48 | 0.623 ± 0.005 | 2.31 ± 0.046 |
| Diethyl ether/Ethyl Acetate (1:1) as organic solvent | 73 | 0.948 ± 0.018 | 2.99 ± 0.169 |
| | 96 | 0.921 ± 0.005 | 4.31 ± 0.347 |
| | 162 | 1.79 ± 0.086 | 5.32 ± 0.156 |
| | 168 | 1.76 ± 0.134 | 5.03 ± 0.582 |

Data from this table allow concluding that both methods are good for the recovery of carotenes. The table also shows that albeit the different yeast species and the differences in their cell wall resistance, the method comprising vortex agitation and extraction with Diethyl ether/Ethyl Acetate (1:1) as organic solvent is the best in terms of carotene concentration. This later particular method is the one proposed and used by the inventors as the reference analytical method for comparing the carotenoid (mainly carotenes) production between yeast species and strains.

Since this method is the one that has been used for the quantification of carotenes in Examples 2-7, it is disclosed in more detail:
Culture medium where the yeast strains grew was centrifuged for 10 min at 10000 rpm and washed. The resulting pellet was re-suspended in DMSO (pre-heated at 50 °C) to cause disruption of yeast cell-wall. Carotenoids were extracted with a solvent mixture of diethyl ether/ethyl acetate (1/1 v/v) and agitated in a GenoGrinder® for 5 min at 480 min⁻¹. Extraction was performed twice and supernatants were finally mixed. NaCl was added to promote phase-separation during centrifugation (10 min, 10000 rpm).

A known volume of this upper phase was evaporated with nitrogen flow. Extracted carotenoids were dissolved in acetone (4 ml), the dissolution was filtered (0.45 µm) and the absorbance measured at 454 nm (UV-Vis spectrophotometer). Calibration pattern was performed with β-carotene dilutions (0.2-10 µg/ml). With this procedure concentration of carotenes is determined/quantified by UV-Vis as β-carotene equivalents at 450 nm. As above exposed the "β-carotene equivalents" is the total amount of carotenes of any type in a solution that give the same absorbance units (A.U.) that will correspond to a solution with pure β-carotene (with no other carotene type compound).

For industrial purposes, in particular due to food industry regulations, cell wall disruption and carotene extraction cannot be performed with DMSO. Therefore, alternative methods have been assayed, particularly the combination of mill or high-voltage pulses cell disruption with the extraction with acetone as organic solvent.

Next table 7, relating to *Sporobolomyces roseus* CECT 13123, details the recovery of carotenes (mg/g of biomass) obtained in two fermentation assays (1 and 2) and using different recovery procedures.

**Table 7. Comparison of carotene recovery from Sporobolomyces roseus CECT 13123 (quantified by UV-Vis as beta-carotene equivalents at 450 nm) (mg/g biomass)**

| FERMENTATION EXPERIMENT | RECOVERY PROCEDURE | | |
|---|---|---|---|
| | DMSO (50 °C) and vortex agitation | Mill | High-voltage |
| | | Acetone as organic solvent | Electric Pulses |
| | | | Acetone as organic solvent |
| | Diethyl ether/Ethyl Acetate (1:1) as organic solvent | | |
| 1 | 0.065 | 0.075 | 0.091 |
| 2 | 0.043 | 0.050 | 0.059 |

This table 7 allows concluding that when scaling the lab procedures (DMSO cell wall disruption), the recovery is maintained or even improved.

### Example 9. Identification of carotene composition

The total amount of carotene compounds was measured by UV-Vis spectrometry as β-carotene equivalent at 454nm. This method has been used in all the previous examples when referring to quantification of carotenes.

Moreover, the identification and quantification of individual carotene compounds were performed by High Performance Liquid Chromatography (HPLC from Agilent, C18 column, mobile phase gradient of acetone (A) and water (B) at 1 mL/min with an injection volume of 20 microliters) and Diode Array Detection at two different wavelengths (450nm and 490nm). The main compounds identified in each of the assayed samples from fermentation of PDA medium by CECT 13123 were: β-carotene, γ-carotene, torulene and torularhodin. Any reference standard isn't available for torulene quantification; therefore, this compound is quantified using a β-carotene standard according to bibliography.

The results obtained for *Sporobolomyces roseus* CECT 13123 in several fermentations of PDA medium at 168 hours are showed in Table 8, wherein ranges of percentages by weight of each of the detected carotenes were established. The data are also depicted in the chromatogram of FIG. 11.

**Table 8. Carotene composition from Sporobolomyces roseus CECT 13123**

| Carotene compound | Range % (weight/weight, the total of carotenes compounds being 100% of carotenes in the sample) (Quantification by HPLC/ beta-carotene equivalent by UV-Vis measurement) |
|---|---|
| β - carotene | 48 - 50 |
| γ - carotene | 10 - 12 |
| Torulene | 26 - 28 |
| Torularhodin | 12 - 14 |

Total carotene production and the relative composition depend on several aspects, such as the medium composition (i.e. type and concentration of sugar or nitrogen), the stress factors (light, oxygen, etc.), and the fermentation time. This is so, because production of carotenes by means of the yeast strain is in response to stress conditions. Therefore, depending on said stress a quantitative pattern of carotenes is obtained within the ranges of Table 8. β-carotene with at least 40 % by weight is always obtained and the rest of the indicated carotenes are also present in the colored composition obtainable by the action of *Sporobolomyces roseus* CECT 13123.

### Example 10. Isolation of carotenoid fractions

A solid phase extraction (in Silica) was performed to obtain different colorant compositions. One composition was related to torularhodin fraction and other composition was related to β-carotene, torulene and γ-carotene.

As the skilled man will note, β-carotene can be further purified from the rest of the carotenes (torulene and γ-carotene) if desired, by means of several distillation series, or alternatively by several solvent extraction (solid phase or liquid-liquid extraction) steps, or alternatively by chromatography techniques.

### REFERENCES CITED IN THE APPLICATION

- Marova et al. "Use of several waste substrates for carotenoid-rich biomass yeast production", Journal of Environmental Management-2012, vol. no. 95, pp.:s338-s342
- Mata-Gómez et al. "Biotechnological production of carotenoids by yeast: an overview", Microbial Cell Factories-2014, vol. no. 13:12.
- US20120142082
- JPH0622748
- WO2011130576

## Claims

1. An isolated yeast strain of *Sporobolomyces roseus* deposited on the 25th March 2015 at the Colección Española de Cultivos Tipo (CECT) with the identification reference LGO1107 and which received the accession number CECT 13123.

2. A process for obtaining a colored composition comprising carotenoids, the process comprising:
- fermenting under aerobic conditions a culture medium composition comprising a carbon and a nitrogen source with a yeast strain as defined in claim 1;
- isolating the yeast strain; and
- disrupting yeast cell-wall to deliver the composition comprising carotenoids.

3. The process according to claim 2, comprising the steps of:
(a) preparing a pre-culture of a strain as defined in claim 1;
(b) inoculating a culture medium composition comprising a carbon and a nitrogen source with the pre-culture of step (a);
(c) aerobically fermenting the carbon source of the culture medium composition; and
(d) isolating the yeast strain ,and
(e) disrupting yeast cell-wall to deliver the composition comprising carotenoids.

4. The process according to claim 3, wherein step (b) is carried out with a strain inoculum concentration from 10³ to 10⁸ CFU/ml of culture medium.

5. The process according to any of claims 2-4, wherein fermenting the culture medium is performed in a temperature range from 20 °C to 35 °C.

6. The process according to any of claims 2-5, wherein fermenting the culture medium is carried out in a pH range from 2.0 to 8.0.

7. The process according to any of claims 2-6, wherein aerobic conditions comprise oxygenation at a rate flow of 2 to 10 L/min from 20 %v/v-30 % v/v, wherein v/v means volume of air containing until 21 % of O₂ per volume of culture medium of culture medium.

8. The process according to any of claims 2-7, wherein the culture medium composition comprising a carbon and a nitrogen source is a culture medium comprising citrus fruit molasses.

9. A colored composition comprising carotenoids obtainable by a process as defined in any of claims 2-8, said composition comprising β-carotene, γ-carotene, torulene and toluarhodin.

10. The colored composition according to claim 9, wherein the percentage by weight of β-carotene is of at least 40 % in relation to the total amount of carotenes in the composition (w/w), said carotenes recovered by disrupting yeast cells with dimethyl sulfoxide pre-heated at 50 °C, and extracting the disrupted yeast cell with diethyl ether/ethyl acetate (1:1).

11. Use of a strain as defined in claim 1, or of a colored composition as defined in any of claims 9-10 as antioxidant agents.

12. Use of a strain as defined in claim 1, or of a colored composition as defined in any of claims 9-10, as coloring producing agent.

13. The use according to claim 12, wherein the coloring effect is applied on a product selected from the group consisting of an edible product, an oral pharmaceutical composition, a nutraceutical composition and a cosmetic composition.

14. An edible product which comprises an effective amount of a strain as defined in claim 1, or a colored composition as defined in any of claims 9-10 together with appropriate amounts of edible ingredients.

15. Use of a strain as defined in claim 1, or of a colored composition as defined in any of claims 9-10, as an ingredient of an edible product.
